# EUROPEAN PATENT SPECIFICATION

(11) **EP 1 201 637 B1**
(45) Date of publication and mention of the grant of the patent: **16.03.2005**
(21) Application number: 01309022.0
(22) Date of filing: 24.10.2001
(51) Int. Cl.: C07C 51/27, C07C 51/285, C07C 51/29, C07C 57/10, C07C 57/08, C07C 57/04, C07C 57/44

(54) **Process for the preparation of unsaturated carboxylic acids**
Verfahren zur Herstellung von ungesättigten Carbonsäuren
Procédé pour la production d'acides carboxyliques insaturés

(30) Priority: 25.10.2000 IN MU09572000
(43) Date of publication of application: 02.05.2002
(73) Proprietor: Somaiya Organo Chemicals Limited, Mumbai 400001 (IN)
(72) Inventor: Chandalia, S.B., Mumbai 400058 (IN); Chandnani, Kavita, Andheri (West), Mumbai 400058 (IN); Srivastava, Sangeeta, Colab, Mumbai 400005 (IN)
(74) Representative: Campbell, Neil Boyd

(56) References cited:
- EP-A- 0 571 864
- GB-A- 742 282
- GB-A- 1 520 818
- US-A- 3 361 805
- DATABASE WPI Section Ch, Week 197642 Derwent Publications Ltd., London, GB; Class A41, AN 1976-78485X XP002224871 & JP 51 100018 A (SUMITOMO CHEM CO LTD), 3 September 1976 (1976-09-03)
- DATABASE WPI Section Ch, Week 198221 Derwent Publications Ltd., London, GB; Class A60, AN 1982-42240E XP002224872 & JP 57 062238 A (DAICEL CHEM INDS LTD), 15 April 1982 (1982-04-15)

## Description

The present invention relates to a process for the manufacture of unsaturated carboxylic acids or salts thereof, in particular α,β-unsaturated carboxylic acids such as sorbic acid or salts thereof by the oxidation of the corresponding aldehyde. In particular, oxidation takes place in the presence of a transition metal oxide or hydroxide catalyst using an oxidising agent selected from sodium hypohalites, hypohalous acids, peroxides or peracids.

Sorbic acid (2,4-hexadienoic acid) and its salts are used as preservatives for foods because of their excellent antifungal activity. Various processes are known for the preparation of sorbic acid, e.g. the condensation of malonic acid with crotonaldehyde, the reaction of crotonaldehyde with a zinc derivative of an acetic-bromine ester (as per the Reformatzky synthesis), oxidisation of hexadienal with silver salts, the oxidation of 3,5-heptadienone with sodium hypochlorite and others.

Sorbic acid has been industrially manufactured by reacting crotonaldehyde with ketene to give a polyester (via a lactone produced as an intermediate) and hydrolysing the polyester with an alkali or acid or by using an ion exchange resin. However, the above-mentioned process requires a high input of energy during the dehydration of acetic acid to ketene and it is not economically viable. Furthermore, recovery and purification of the sorbic acid entails several steps and involves complicated process control.

Unsaturated organic acids are also prepared by the oxidation of corresponding aldehydes with air. As the aldehydes are unsaturated, the oxidation frequently becomes difficult because of the formation of byproducts, which reduce the yield of the end product and adversely affect the purity thereof.

US Patent No. 3,313,843 describes the conversion of ethylenic aldehydes into corresponding acids using nickel peroxide in an alcoholic medium. However, the use of a stoichiometric amount of nickel peroxide would not be cost effective. US Patent No. 2,887,496 and Indian Patent Specification No. 139231 describe the oxidation of aldehydes with air, in the presence of a metal catalyst such as silver and an aqueous solution of alkaline reagents. In this case, the desired acid is obtained in the form of an alkali metal salt and one has to thereafter acidify the salt to liberate the actual acid.

The yield of crude acid is good but the amount of catalyst employed is relatively large and the acid obtained is a crude product which must be purified by washing, recrystallisation and drying under reduced pressure. French Patent No. 1,427,301 describes the oxidation of unsaturated aldehydes with air in the presence of a palladium salt and a copper halide or iron halide.

It is a well-known fact that expenditure is high when molecular oxygen is used as an oxidant. Furthermore, under the conditions used, the oxidation of hexadienal is accompanied by many side reactions resulting in polymeric by products. The use of molecular oxygen as an oxidising agent therefore does not produce the desired product.

Indian Patent Specification No. 139231 shows that air oxidation of aldehyde results in a lot of by products and yield is poor (54% to 74%). During oxidation another isomer of sorbic acid, i.e. cis-2-trans-4-hexadienoic acid is formed which is difficult to convert into trans-2-trans-4 hexadienoic acid. For these reasons, the above processes are expensive, uneconomical and poor in yield.

The object of the present invention is to provide a process for the manufacture of sorbic acid or a salt thereof and may be extended to other unsaturated aldehydes/acids. The oxidation reaction takes place using reagents such as hydrogen peroxide or alkyl peroxide in the presence of metal oxides or hydroxides as catalysts.

This is a commercially viable process for producing sorbic acid from sorbaldehyde (2,4-hexadienal) in high yield and with a high degree of purity.

Thus, viewed from one aspect the invention provides a process for the manufacture of an unsaturated carboxylic acid or salt thereof comprising oxidising the corresponding unsaturated aldehyde with an oxidising agent selected from alkali metal hypohalites, hypohalous acids, peroxides, pyridine N-oxide or peracids in the presence of a transition metal oxide or hydroxide catalyst.

The transition metal in the oxide or hydroxide catalyst is preferably selected from manganese, nickel, copper, silver, platinum, palladium or cobalt. In a preferred embodiment, the transition metal is silver. In general, it was found that elements from groups 9, 10 and 11 (i.e. those groups headed by cobalt, nickel and copper) were advantageous. The transition metal may be in any convenient oxidation state and this state varies depending on the nature of the transition metal and the relative stability of its oxides or hydroxides. In general, the most stable oxide or hydroxide of a transition metal is favoured.

Preferably, the catalyst should be a transition metal oxide. Hence, a particularly favoured catalyst is silver (I) oxide (Ag₂O).

Any unsaturated aldehyde may be oxidised by the process of the invention although the invention is particularly suited to the oxidation of α,β-unsaturated aldehydes, especially α,β,γ-unsaturated aldehydes. Specific aldehydes suitable for oxidation by the process of the invention include sorbaldehyde, acrolein, methaacrolein, crotonaldehyde, cinnamaldehyde, and homologues thereof. The process is especially suitable for the formation of sorbic acid or salts thereof.

The oxidising agent of use in the invention may be an alkali metal hypohalite salt, such as a hypochlorite, e.g. sodium hypochlorite, or a hypohalous acid or salt thereof, e.g. a lithium, sodium or calcium salt thereof. Other suitable oxidising agents include peroxides such as benzoyl peroxide, hydrogen peroxide, acetyl peroxide and alkyl hydrogen peroxides such as amyl hydroperoxide. Peracids such as peracetic acid, perbenzoic acid, mono perthalic acid or m-chloro perbenzoic acid also give good yields. Finally, pyridine N-oxide is also a suitable oxidising agent.

Particularly preferred oxidising agents are hydrogen peroxide, alkyl hydroperoxide and acylperoxide since it has been surprisingly found that the conversion to carboxylic acid and the selectivity of said conversion is high giving rise to few impurities and hence allowing ready purification. The yield of carboxylic acid is also high. Of these oxidising agents, hydrogen peroxide is preferred.

It is desirable to keep the oxidation reaction at a basic pH, i.e. a pH greater than seven. This may be achieved by adding any inert basic reagent into the reactor. In one embodiment, the transition metal hydroxide may act as both basic reagent and catalyst. However, it is preferred that an alkali metal or alkaline earth metal hydroxide is used to maintain basicity. Suitable reagents include calcium hydroxide, potassium hydroxide, sodium hydroxide, etc. Ammonium hydroxides may also be used. The ratio of hydroxide to unsaturated aldehyde may preferably be 1:1.2.

The inorganic alkaline reagent used in the reaction should be employed as part of an aqueous solution. The molar ratio discussed above is sufficient to convert the acid produced during the oxidation reaction into the salt at a rate at which it is formed.

As an alternative to an alkali hydroxide it is possible to use sodium carbonate or any suitable organic base such as amine, e.g. triethanol amines, mono, dialkyl amines, EDTA, etc. since these may reduce the formation of undesirable polymeric side products.

The oxidation process should advantageously take place in an aqueous alcohol solution e.g. a solution of methanol, ethanol, propanol, butanol, pentanol or decanol. It has surprisingly been found that the use of an alcohol solution enhances reaction rates. Preferably, the oxidation reaction is maintained at a temperature between 5 to 80°C, preferably between 20 to 40°C.

The reaction is conventionally carried out over a period of 4 to 24 hours with the aldehyde and catalyst components being held in a suitable solvent whilst the oxidising agent and alkali component are added dropwise.

By using the process as hereinbefore described, it is possible to obtain yields of 92 to 98% unsaturated carboxylic acids. Since the reaction is maintained at a basic pH the acid is initially formed as a salt. The pure acid is, of course, liberated upon simple acidification of the corresponding salt with any conventional strong acid, e.g. hydrochloric acid.

The invention will now be described further in relation to the following non-limiting examples.

### Example 1

500 ml of water and 100 g of 2,4-hexadienal containing 5 g of cobalt (II) oxide were placed in a 1000 cm³ reactor. The reactor contents were stirred at 1200 rpm at 50°C. 200 ml of 12% sodium hypochlorite and 80 g of sodium carbonate were added over a period of 10 hours. The sodium salt of sorbic acid resulted.

Better yields however were obtained by generating cobalt (II) oxide *in situ* in the reactor rather than adding this directly to the reactor. In this embodiment, the catalyst was prepared by reacting cobalt (II) nitrate with equimolar amounts of sodium hypochlorite. This mixture was continuously stirred. The resulting solution was filtered and after filtration, acidified with 20% hydrochloric acid. The yield or sorbic acid was 72.9 g corresponding to 94% yield.

### Example 2

500 ml water and 300 ml ethanol were mixed and added to a reactor along with 100 g of sorbaldehyde with 2% w/v catalyst loading of copper (II) oxide. 50 g of benzoyl peroxide and 59 g potassium hydroxide were added at 42°C over a period of eight hours. The reaction was worked up as per Example 1 to yield 84 g of sorbic acid at 95.2% yield.

### Example 3

300 ml water and 200 ml of propanol were mixed and added to a reactor along with 100 g of sorbaldehyde with 5% w/v nickel (IV) oxide. 50 g of benzoyl peroxide and 77 g potassium hydroxide were added at 42°C over a period of eight hours. Work-up was conducted as per Example 1 to yield 81 g of sorbic acid.

In order to improve yield, nickel (IV) oxide was prepared *in situ* in the reactor by the combination of nickel (IV) sulfate and sodium hydroxide which improved yields by 5%.

### Example 4

475 ml water and 25 ml methanol were mixed and added to a reactor along with 100 g or sorbaldehyde with a catalyst loading of 10% w/v manganese (IV) oxide. 50 g of benzoyl peroxide and 50 g potassium hydroxide were added at 57°C over 5.5 hours. Work up was conducted as in Example 1 to yield 70.6 g of sorbic acid at 97.6% yield.

### Example 5

25 ml water and 475 ml of propanol were mixed and added to a reactor along with 100 g of sorbaldehyde with a catalyst loading of 3% w/v platinum (IV) oxide. 200 ml of hydrogen peroxide and 55 ml of triethanol amine were added separately at 77°C over 12 hours. Work up was conducted as in Example 1 to yield 67.6 g of sorbic acid at 94.6% purity.

### Example 6

500 ml of water and 100 g of sorbaldehyde with 5% w/v nickel (IV) oxide were added to a reactor. 90g of pyridine N-oxide was added. Pyridine liberated during the reaction acts as a base. Acidification of the sorbic acid salt was achieved using 30% HCl to yield 74 g of sorbic acid at 92.3% purity.

### Example 7

500 ml water and 100 g of sorbaldehyde with 5% w/v silver (I) oxide were added to a reactor. 90 g of hydrogen peroxide was also added.

Yield 85 g of sorbic acid at 99.3% purity.

## Claims

1. A process for the manufacture of an unsaturated carboxylic acid or salt thereof comprising oxidising the corresponding unsaturated aldehyde with an oxidising agent selected from alkali metal hypohalites, hypohalous acids, peroxides, pyridine N-oxide or peracids in the presence of a transition metal oxide or hydroxide catalyst.

2. A process as claimed in claim 1 wherein the transition metal in the oxide or hydroxide catalyst is selected from manganese, nickel, copper, silver, platinum, palladium or cobalt.

3. A process as claimed in claim 2 wherein the transition metal is silver.

4. A process as claimed in any one of claims 1 to 3 wherein the catalyst is a transition metal oxide.

5. A process as claimed in any one of claims 1 to 4 wherein said unsaturated aldehyde is an α,β-unsaturated aldehyde.

6. A process as claimed in claim 5 wherein said α,β-unsaturated aldehyde is sorbaldehyde, acrolein, methaacrolein, crotonaldehyde or cinnamaldehyde.

7. A process as claimed in claim 6 wherein said α,β-unsaturated aldehyde is sorbaldehyde.

8. A process as claimed in any one of claims 1 to 7 wherein said oxidising agent is sodium hypochlorite, hydrogen peroxide, benzoyl peroxide or pyridine N-oxide.

9. A process as claimed in any one of claims 1 to 8 wherein the reaction takes place at a basic pH.

10. A process as claimed in claim 9 wherein said basic pH is achieved by the addition of calcium hydroxide, potassium hydroxide, sodium hydroxide or ammonium hydroxide to the reaction medium.

11. A process as claimed in claim 10 wherein the ratio of hydroxide to unsaturated aldehyde is 1:1.2.

12. A process as claimed in any one of claims 1 to 11 wherein the reaction takes place in an aqueous alcohol solution.

13. A process as claimed in any one of claims 1 to 12 wherein the oxidation reaction is maintained at a temperature between 20 to 40°C.

## Patentansprüche

1. Verfahren zur Herstellung einer ungesättigten Carbonsäure oder eines Salzes davon, umfassend das Oxidieren des entsprechenden ungesättigten Aldehyds mit einem aus Alkalimetallhypohalogeniten, hypohalogenigen Säuren, Peroxiden, Pyridin-N-Oxid oder Persäuren ausgewählten Oxidationsmittel in Gegenwart eines Übergangsmetalloxid- oder -hydroxidkatalysators.

2. Verfahren nach Anspruch 1, wobei das Übergangsmetall in dem Oxid- oder Hydroxidkatalysator ausgewählt ist aus Mangan, Nickel, Kupfer, Silber, Platin, Palladium oder Kobalt.

3. Verfahren nach Anspruch 2, wobei das Übergangsmetall Silber ist.

4. Verfahren nach einem der Ansprüche 1 bis 3, wobei der Katalysator ein Übergangsmetalloxid ist.

5. Verfahren nach. einem der Ansprüche 1 bis 4, wobei der ungesättigte Aldehyd ein α,β-ungesättigter Aldehyd ist.

6. Verfahren nach Anspruch 5, wobei der α,β-ungesättigte Aldehyd Sorbinaldehyd, Acrolein, Methacrolein, Crotonaldehyd oder Zimtaldehyd ist.

7. Verfahren nach Anspruch 6, wobei der α,β-ungesättigte Aldehyd Sorbinaldehyd ist.

8. Verfahren nach einem der Ansprüche 1 bis 7, wobei das Oxidationsmittel Natriumhypochlorit, Wasserstoffperoxid, Benzoylperoxid oder Pyridin-N-oxid ist.

9. Verfahren nach einem der Ansprüche 1 bis 8, wobei die Reaktion bei einem basischem pH stattfindet.

10. Verfahren nach Anspruch 9, wobei der basische pH durch Zugabe von Kalziumhydroxid, Kaliumhydroxid, Natriumhydroxid oder Ammoniumhydroxid zum Reaktionsmedium erreicht wird.

11. Verfahren nach Anspruch 10, wobei das Verhältnis von Hydroxid zu ungesättigtem Aldehyd 1:1,2 beträgt.

12. Verfahren nach einem der Ansprüche 1 bis 11, wobei die Reaktion in einer wässrigen Alkohollösung stattfindet.

13. Verfahren nach einem der Ansprüche 1 bis 12, wobei die Oxidationsreaktion bei einer Temperatur zwischen 20 bis 40 °C gehalten wird.

## Revendications

1. Procédé pour la fabrication d'un acide carboxylique insaturé ou de l'un de ses sels comprenant l'oxydation de l'aldéhyde insaturé correspondant par un agent oxydant choisi parmi des hypohalogénites de métaux alcalins, des acides hypohalogéneux, des peroxydes, du N-oxyde de pyridine ou des peracides en présence d'un catalyseur oxyde ou hydroxyde de métal de transition.

2. Procédé revendiqué dans la revendication 1, dans lequel le métal de transition dans le catalyseur oxyde ou hydroxyde est choisi parmi le manganèse, le nickel, le cuivre, l'argent, le platine, le palladium ou le cobalt.

3. Procédé revendiqué dans la revendication 2, dans lequel le métal de transition est l'argent.

4. Procédé revendiqué dans l'une quelconque des revendications 1 à 3, dans lequel le catalyseur est un oxyde de métal de transition.

5. Procédé revendiqué dans l'une quelconque des revendications 1 à 4, dans lequel ledit aldéhyde insaturé est un aldéhyde α,β-insaturé.

6. Procédé revendiqué dans la revendication 5, dans lequel ledit aldéhyde α,β-insaturé est le sorbaldéhyde, l'acroléine, la méthaacroléine, le crotonaldéhyde ou le cinnamaldéhyde.

7. Procédé revendiqué dans la revendication 6, dans lequel ledit aldéhyde α,β-insaturé est le sorbaldéhyde.

8. Procédé revendiqué dans l'une quelconque des revendications 1 à 7, dans lequel ledit agent oxydant est l'hypochlorite de sodium, le peroxyde d'hydrogène, le peroxyde de benzoyle ou le N-oxyde de pyridine.

9. Procédé revendiqué dans l'une quelconque des revendications 1 à 8, dans lequel la réaction a lieu à pH basique.

10. Procédé revendiqué dans la revendication 9, dans lequel ledit pH basique est obtenu par l'addition d'hydroxyde de calcium, d'hydroxyde de potassium, d'hydroxyde de sodium ou d'hydroxyde d'ammonium au mélange réactionnel.

11. Procédé revendiqué dans la revendication 10, dans lequel le rapport de l'hydroxyde à l'aldéhyde insaturé est égal à 1/1,2.

12. Procédé revendiqué dans l'une quelconque des revendications 1 à 11, dans lequel la réaction a lieu dans une solution aqueuse d'alcool.

13. Procédé revendiqué dans l'une quelconque des revendications 1 à 12, dans lequel la réaction d'oxydation est maintenue à une température comprise entre 20 et 40 °C.
